(19) Europäisches Patentamt
European Patent Office
Office européen des brevets

(11) Veröffentlichungsnummer: **0 257 333**
**A1**

(12) **EUROPÄISCHE PATENTANMELDUNG**

(21) Anmeldenummer: **87111014.4**

(22) Anmeldetag: **29.07.87**

(51) Int. Cl.⁴: **C 07 D 241/46**, C 07 D 403/12 // A61K31/495

(30) Priorität: **12.08.86 DE 3627310**

(43) Veröffentlichungstag der Anmeldung: **02.03.88 Patentblatt 88/9**

(84) Benannte Vertragsstaaten: **CH DE FR GB LI NL SE**

(71) Anmelder: **Gesellschaft für Biotechnologische Forschung mbH (GBF), Mascheroder Weg 1, D-3300 Braunschweig-Stöckheim (DE)**

(72) Erfinder: **Morr, Michael, Dr., Elmblick 53, D-3340 Wolffenbüttel (DE)**
Erfinder: **Kakoschke, Christel, Kampstrasse 1, D-3153 Lahstadt 2 (Adenstedt) (DE)**
Erfinder: **Tsai, Hsin, Dr., Am Schiffhorn 15, D-3300 Braunschweig (DE)**
Erfinder: **Getzlaff, Rita, Vallstedter Weg 4, D-3303 Vechelde (DE)**

(74) Vertreter: **Boeters, Hans Dietrich, Dr. et al, Boeters, Bauer & Partner Thomas-Wimmer-Ring 14, D-8000 München 22 (DE)**

(54) **Pyocyanin-Derivate und Herstellungsverfahren.**

(57) Die Erfindung betrifft Pyocyanin-Derivate mit einer reaktiven oder reaktivierbaren Seitenkette in 7- oder 8-Position des Phenazinringes, die man an aminogruppenhaltige wasserlösliche oder wasserunlösliche Polymere koppeln kann. Ausserdem betrifft die Erfindung ein Verfahren zur Herstellung dieser Pyocyanin-Derivate.

EP 0 257 333 A1

Pyocyanin-Derivate und Herstellungsverfahren

Pyocyanin ist ein blaues Pigment, das von Pseudomonas aeruginosa gebildet wird. Obgleich seit langem bekannt ist, daß Pyocyanin gegenüber gram-positiven und gram-negativen Bakterien bakterizid wirkt, ist Pyocyanin bisher nicht auf dem therapeutischen Sektor verwendet worden. Der Grund dafür dürfte in der cytotoxischen Wirkung auf Tierzellen zu sehen sein.

Die in-vitro-Cytotoxizität des Pyocyanins konnte bei der Erfindung vorausgehenden Arbeiten mit Human-Fibroblasten, Human-Lymphozyten und Maus-Lymphozyten in Kultur bestätigt werden. Daraus ergibt sich, daß die Cytotoxizität des Pyocyanins weder zell- noch artspezifisch ist. Um Pyocyanin für therapeutische Antikrebsmittel verwenden zu können, haben sich nun die Erfinder die Aufgabe gestellt, Pyocyanin zu derivatisieren, um es covalent an aminogruppenhaltige wasserlösliche oder wasserunlösliche Polymere oder Oligomere zu binden, beispielsweise Zellulose, Dextran, Dextranderivate (wie Sephadex) oder Proteine, wie tumorspezifische

(da) zum Pyocyanin-Derivat (IXa, IXb) der allgemeinen Formel gemäß Anspruch 1 mit

$R^1 = -C_{1-10}$-Alkylen-$CO_2H$ und

$R^2 = C_{1-2}$-Alkyl

alkyliert oder

(db) mit N-Hydroxy-succinimid oder N-Hydroxy-glutarsäureimid umsetzt und Pyocyanin-Derivate (VIIa, VIIb) der allgemeinen Formel gemäß Anspruch 1 mit

$R^1 = -C_{1-10}$-Alkylen-$CO_2$- 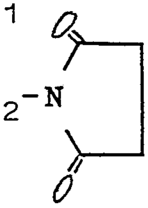

$R^2$ = H (VIIa, VIIb)

gewinnt und gegebenenfalls

(e) das erhaltene Produkt (VIIa, VIIb) zu Pyocyanin-Derivaten der allgemeinen Formel gemäß Anspruch 1 mit

$R^1 = -C_{1-10}$-Alkylen-$CO_2$ 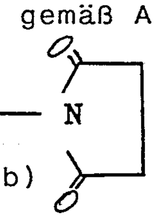

$R^2 = C_{1-2}$-Alkyl (VIIIa, VIIIb)

alkyliert.

alkyliert oder

(db) mit N-Hydroxy-succinimid oder N-Hydroxy-glutarsäureimid umsetzt und Pyocyanin-Derivate der allgemeinen Formel gemäß Anspruch 1 mit

$R^1 = -C_{1-10}$-Alkylen-$CO_2$- 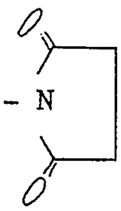

$R^2$ = H (VIIa, VIIb) .

gewinnt und gegebenenfalls

(e) das erhaltene Produkt (VIIa, VIIb) zu Pyocyanin-Derivaten der allgemeinen Formel gemäß Anspruch 1 mit

$R^1 = -C_{1-10}$-Alkylen-$CO_2$ 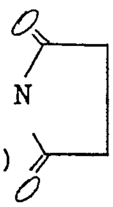

$R^2 = C_{1-2}$-Alkyl (VIIIa, VIIIb)

alkyliert.

Die Verseifung der Stufe (c) kann man bei dem Produkt (Va, Vb) schonend mit Esterase durchführen.

Die erfindungsgemäßen Pyocyanin-Derivate sind 1:1-Gemische von Stellungsisomeren, die in 7- bzw. 8-Position des Phenazinringes substituiert sind. Die Stellungsisomeren lassen sich gaschromatographisch trennen. Für ihre Weiterverwendung scheint eine Trennung jedoch nicht erforderlich zu sein, da die beiden Stellungsisomeren jeweils das für die antibiotische Wirkung gegenüber gram-positiven und gram-negativen Bakterien verantwortliche Strukturelement gemeinsam besitzen.

Nachstehend wird die Erfindung durch Beispiele näher erläutert.

4'-(1-Methoxy-phenazin-7/8-yl)-buttersäuremethylester (3a, 3b)

2.2 g (10.6 mmol) 4'-(3,4-Diaminophenyl)-buttersäuremethylester (1) werden in 14.1 ml Eisessig und 35 ml wasserfreiem Benzol gelöst und schnell zu einer Lösung von 2 g (14.5 mmol) 3-Methoxyo-chinon (2) in 700 ml Benzol getropft. Nach etwa 5 h wird der Reaktionsansatz dreimal mit je 200 ml Wasser, zweimal mit je 100 ml ges. Natriumhydrogencarbonat-Lösung und zweimal mit je 200 ml Wasser ausgeschüttelt. Die gelbe benzolische Lösung wird mit Natriumsulfat getrocknet und zur Trockne eingeengt.
Ausbeute: 2.8 g (85 % d. Th.) an Rohprodukt.

Die Feinreinigung erfolgte über eine Kieselgelsäule (39 x 5 cm) und stufenweise Elution mit Dichlormethan und Dichlormethan/Methanol-Mischungen 98.5:1.5/95:5/92.5:7.5.
Ausbeute: 2.04 g (62 % d. Th.).
Die Stellungsisomeren 3a und 3b sind gaschromatographisch auf einer Kapillarsäule mit Methylsiliconphase trennbar.
GC-MS (Gaschromatograph-Massenspektrum) ergab eine Masse von 310 ($C_{18}H_{18}N_2O_3$ M.W. = 310.35).

4'-(1-Hydroxy-phenazin-7/8-yl-buttersäuremethylester (4)

1.2 g (3.87 mmol) 3a, 3b werden in ca. 250 ml trockenem Benzol gelöst und unter Rühren und Feuchtigkeitsausschluß mit einer Lösung von ca. 6 g wasserfreiem Aluminiumbromid in 200 ml Benzol versetzt. Nach etwa 3 h wird die braune Lösung zur Trockne eingeengt und vorsichtig mit Eis und Essigester versetzt. Die violette wäßrige Lösung (pH 3.3) wird fünfmal mit je 50 ml Essigester extrahiert. Der grün-gelbe Essigester-Extrakt wird mit Natriumsulfat getrocknet und zur Trockne eingeengt. Man erhält 1.01 g 4 (88 % d. Th.).

Die wäßrige violette Lösung wird auf pH 9 eingestellt und mit Chloroform extrahiert. Der blaue Chloroformextrakt wird mit salzsaurem Wasser überschichtet und geschüttelt. Die violette wäßrige Lösung wird abgetrennt, erneut auf pH 9 eingestellt und mit Chloroform das blaue Nebenprodukt 5 extrahiert.
Ausbeute: 0.14 g 5.

4'-(1-Hydroxy-phenazin-7/8-yl)-buttersäure (6)

3 g (10 mmol) 4 werden in 50 ml 25 %iger methanolischer Kalilauge gelöst und 3 h bei Raumtemperatur stehengelassen. Die rotviolette Lösung wird mit 10 %iger Salzsäure im Eisbad auf pH 4 eingestellt (Gelbfärbung). Nach dem Abziehen des Methanols im Rotavapor wird die wäßrige Lösung fünfmal mit je 60 ml Essigester ausgeschüttelt. Nach dem Trocknen mit Natriumsulfat und Einengen zur Trockne erhält man etwa 2.55 g (89 % d. Th.) 6.

$C_{16}H_{14}N_2O_3$ (282.3)

| | % C | % H | % N |
|---|---|---|---|
| Ber. | 68.07 | 5.00 | 9.92 |
| Gef. | 67.87 | 5.11 | 10.13 |

4'-(1-Hydroxy-phenazin-7/8-yl)-buttersäure-N-hydroxysuccinimidester (7)

0.5 g (1.8 mmol) 6 werden in 130 ml wasserfreiem Essigester gelöst und mit 214 mg (1.86 mmol) N-Hydroxysuccinimid und 384 mg (1.86 mmol) Dicyclohexylcarbodiimid (DCC) versetzt. Man läßt über Nacht rühren, filtriert vom ausgefallenen Dicyclohexylharnstoff ab und engt zur Trockne ein. Das nicht ganz saubere Produkt (≈0.6 g) wird für weitere Umsetzungen in wasserfreiem Dimethylformamid gelöst und im Kühlschrank gelagert.

4'-(1-Keto-5(N)-methylphenazin-7/8-yl)-buttersäure-N-hydroxy-succinimidester (8)

60 mg (≈0.16 mmol) 7 werden mit 2 ml frisch destilliertem Dimethylsulfat versetzt und 10 min auf 100°C erhitzt. Anschließend wird in Eis gekühlt und mit Ether versetzt. Der braune Niederschlag wird über eine Fritte abgesaugt, mit Ether gewaschen und in ges. Natriumhydrogencarbonat-Lösung gelöst. Man schüttelt mit Chloroform aus, wäscht die blaue Chloroformphase mit salzsaurem Wasser (pH 3). Die rot-violette Wasserphase wird abgetrennt, mit ges. Natriumhydrogencarbonat-Lösung alkalisiert und erneut mit Chloroform ausgeschüttelt. Man trocknet mit Natriumsulfat und erhält nach dem Einengen zur Trockne etwa 10 mg (≈16 % d. Th.) 8.

4'-(1-Keto-5(N)-methylphenazin-7/8-yl)-buttersäure (9)

3 mg 5 werden in 0.5 ml 0.1 M Phosphatpuffer (pH 8) gelöst und mit 5 µl Esterase (Boehringer) versetzt.
Man läßt bei 25°C stehen und verfolgt die Spaltung des Methylesters dünnschichtchromatographisch auf Kieselgel (Laufmittel: $CH_2Cl_2$ : MeOH 9:1). Nach einer Stunde ist die Spaltung vollständig. Es wird auf pH 5 eingestellt, zur Trockne eingeengt und die freie Säure mit wenig Methanol extrahiert.
Auswaage etwa 2 mg 9.

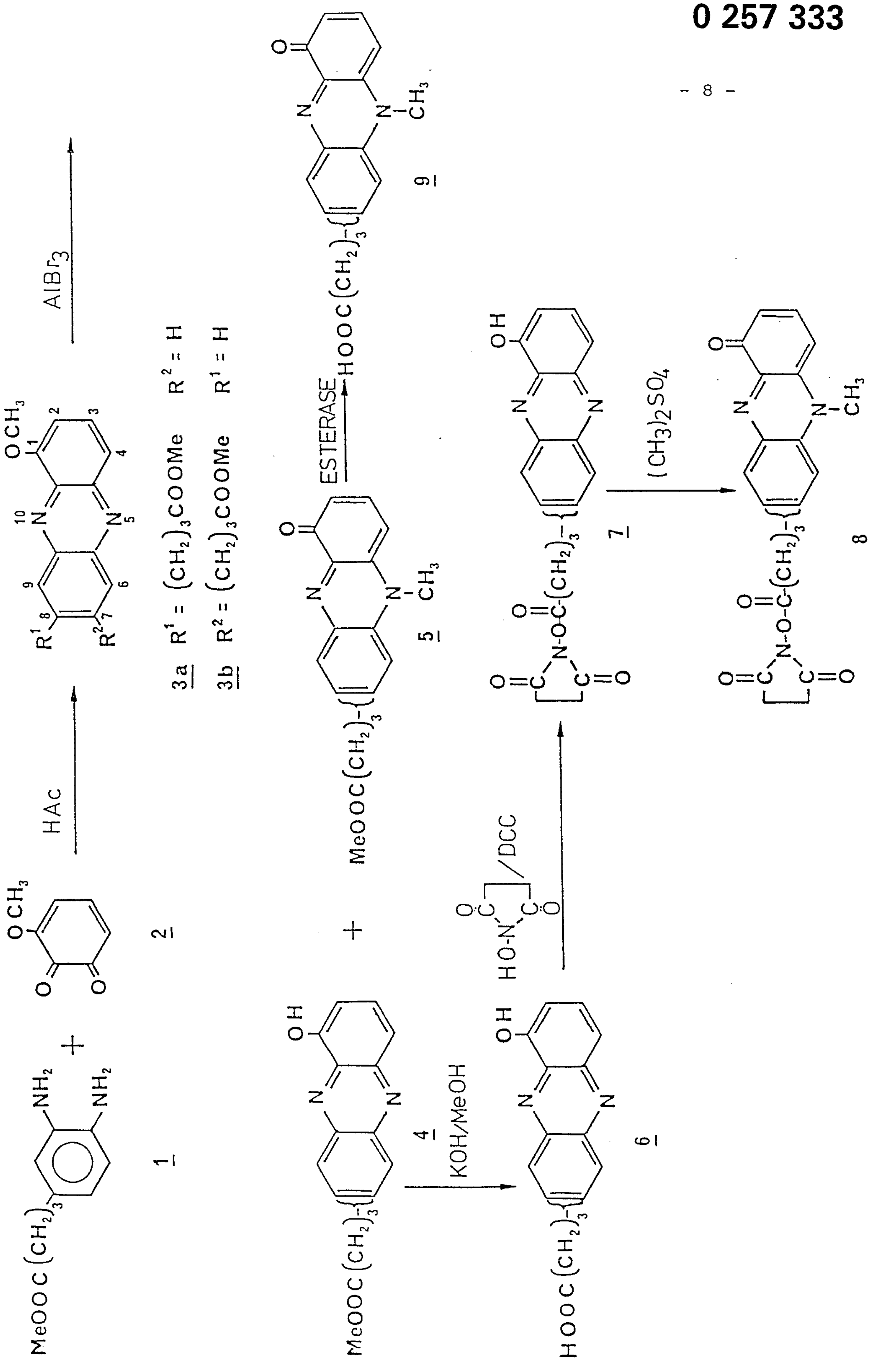


1 2 4 5 6 7 8 9

3a $R^1 = (CH_2)_3COOMe$ $R^2 = H$

3b $R^2 = (CH_2)_3COOMe$ $R^1 = H$

## Patentansprüche

1. Pyocyanin-Derivate der allgemeinen Formel

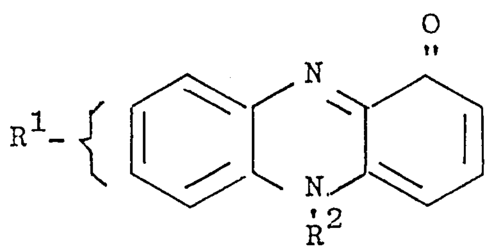

worin

$R^1$ = $-C_{1-10}$-Alkylen-$CO_2$-$C_{1-5}$-alkyl und
$R^2$ = H oder $C_{1-2}$-Alkyl oder

$R^1$ = $-C_{1-10}$-Alkylen-$CO_2H$ und
$R^2$ = H oder $C_{1-2}$-Alkyl oder

$R^1$ = $-C_{1-10}$-Alkylen-$CO_2$- 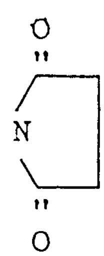
$R^2$ = H oder $C_{1-2}$-Alkyl

sind.

2. Pyocyanin-Derivate nach Anspruch 1, dadurch gekennzeichnet, daß $C_{1-10}$-Alkylen ein Ethylen-, Propylen- oder Butylen-Rest ist.

3. Verfahren zur Herstellung der Verbindungen gemäß Anspruch 1 oder 2, dadurch gekennzeichnet, daß man

(a) omega-(3,4-Diaminophenyl)-$C_{1-10}$-alkansäure-$C_{1-5}$-alkylester (I) mit 3-$C_{1-2}$- Alkoxy-o-chinon (II) zu omega-(1-$C_{1-2}$-Alkoxy-phenazin-7/8-yl)-$C_{1-10}$-Alkansäure-$C_{1-5}$-alkylester (IIIa, IIIb) umsetzt und

(b) das erhaltene Produkt (IIIa, IIIb) mit Aluminiumbromid in einem inerten organischen Lösungsmittel behandelt und Pyocyanin-Derivate der allgemeinen Formel gemäß Anspruch 1 mit

$R^1$ = -$C_{1-10}$- Alkylen-$CO_2$-$C_{1-5}$-alkyl und

$R^2$ = H (IVa, IVb) oder $C_{1-2}$-Alkyl (Va, Vb)

gewinnt und gegebenenfalls

(c) das erhaltene Produkt (IVa, IVb; Va, Vb) zur freien Säure der allgemeinen Formel gemäß Anspruch 1 mit

$R^1$ = -$C_{1-10}$-Alkylen-$CO_2H$ und

$R^2$ = H (VIa, VIb) oder $C_{1-2}$-Alkyl (IXa, IXb)

verseift und gegebenenfalls

(d) die erhaltene freie Säure (VIa, VIb) der allgemeinen Formel gemäß Anspruch 1 mit

$R^1$ = -$C_{1-10}$-Alkylen-$CO_2H$ und

$R^2$ = H

Gegenstand der Erfindung ist ferner ein Verfahren zur Herstellung der erfindungsgemäßen Pyocyanin-Derivate, bei dem man

(a) omega-(3,4-Diaminophenyl)-$C_{1-10}$-alkansäure-$C_{1-5}$-alkylester (I) mit 3-$C_{1-2}$-Alkoxy-o-chinon (II) zu omega-(1-$C_{1-2}$-Alkoxy-phenazin-7/8-yl)-$C_{1-10}$-Alkansäure-$C_{1-5}$-alkylester (IIIa, IIIb) umsetzt und

(b) das erhaltene Produkt (IIIa, IIIb) mit Aluminiumbromid in einem inerten organischen Lösungsmittel behandelt und Pyocyanin-Derivate der allgemeinen Formel gemäß Anspruch 1 mit

$R^1$ = -$C_{1-10}$-Alkylen-$CO_2$-$C_{1-5}$-alkyl und
$R^2$ = H (IVa, IVb) oder $C_{1-2}$-Alkyl (Va, Vb)
gewinnt und gegebenenfalls

(c) das erhaltene Produkt (IVa, IVb; Va, Vb) zur freien Säure der allgemeinen Formel gemäß Anspruch 1 mit
$R^1$ = -$C_{1-10}$-Alkylen-$CO_2H$ und
$R^2$ = H (VIa, VIb) oder $C_{1-2}$-Alkyl (IXa, IXb)
verseift und gegebenenfalls

(d) die erhaltene freie Säure (VIa, VIb) der allgemeinen Formel gemäß Anspruch 1 mit
$R^1$ = -$C_{1-10}$-Alkylen-$CO_2H$ und
$R^2$ = H

(da) zum Pyocyanin-Derivat (IXa, IXb) der allgemeinen Formel gemäß Anspruch 1 mit
$R^1$ = -$C_{1-10}$-Alkylen-$CO_2H$ und
$R^2$ = $C_{1-2}$-Alkyl

Proteine, beispielsweise monoklonale Antikörper.

Dazu werden erfindungsgemäß Pyocyanin-Derivate der folgenden allgemeinen Formel vorgesehen:

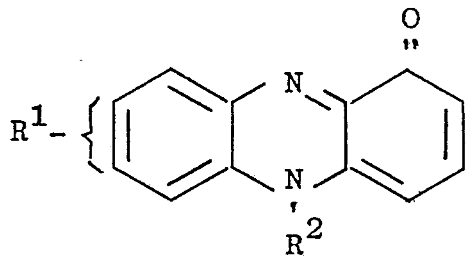

worin

$R^1$ = $-C_{1-10}$-Alkylen-$CO_2$-$C_{1-5}$-alkyl und
$R^2$ = H oder $C_{1-2}$-Alkyl oder

$R^1$ = $-C_{1-10}$-Alkylen-$CO_2H$ und
$R^2$ = H oder $C_{1-2}$-Alkyl oder

$R^1$ = $-C_{1-10}$-Alkylen-$CO_2$- 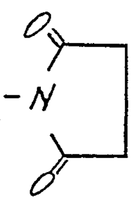
$R^2$ = H oder $C_{1-2}$-Alkyl

sind.

Bei den $C_{1-10}$-Alkylen-Gruppen im Rest $R^1$ kann es sich um einen Ethylen-, Propylen- oder Butylenrest handeln.

Zur Herstellung der erfindungsgemäßen Pyocyanin-Derivate geht man von omega-(3,4-Diaminophenyl)-$C_{1-10}$-alkansäure-$C_{1-5}$-alkylestern (I) aus. 4'-(3,4-Diaminophenyl)-buttersäuremethylester ist bekannt; vgl. Aharoni & Litt, J. Polym. Sci., Polymer Chemistry Edition, 12 (1974) 639. Die anderen omega-(3,4-Diaminophenyl)-$C_{1-10}$-Alkansäure-$C_{1-5}$-alkylester (I), von denen man erfindungsgemäß ausgeht, lassen sich analog zum angegebenen Stand der Technik herstellen.

Europäisches Patentamt

**EUROPÄISCHER RECHERCHENBERICHT**

0 257 333

## EINSCHLÄGIGE DOKUMENTE

EP 87111014.4

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A - 1 670 440 (CANADIAN PATENTS)<br>* Ansprüche 1,5,6,8 *<br>-- | 1,3 | C 07 D 241/46<br>C 07 D 403/12<br>//A 61 K 31/495 |
| A | CHEMICAL ABSTRACTS, Band 82, Nr. 19, 12. Mai 1975, Columbus, Ohio, USA<br>S.B. SEREBRYANYI et al. "Anti-tubercular activity of phenazine derivatives"<br>Seite 51, Spalte 1, Zusammenfassung-Nr. 119 124b<br>& Fiziol. Akt. Veshchestra 1974, 6, 88-92<br>-- | 1 | |
| A | CHEMICAL ABSTRACTS, Band 73, Nr. 19, 9. November 1970, Columbus, Ohio, USA<br>M.E. ORLOWSKI et al. "Effect of chlorpromazine on the growth of bacteria"<br>Seite 110, Spalte 1, Zusammenfassung-Nr. 95 730g<br>& Develop. Ind. Microbiol. 1968, (Pub. 1969) 10, 279-83<br>---- | 1 | **RECHERCHIERTE SACHGEBIETE (Int. Cl.4)**<br>C 07 D 241/00<br>C 07 D 403/00 |

Der vorliegende Recherchenbericht wurde für alle Patentansprüche erstellt.

| Recherchenort | Abschlußdatum der Recherche | Prüfer |
|---|---|---|
| WIEN | 18-11-1987 | LUX |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82